(19) Europäisches Patentamt
European Patent Office
Office européen des brevets

(11) EP 0 888 410 B1

(12) **EUROPÄISCHE PATENTSCHRIFT**

(45) Veröffentlichungstag und Bekanntmachung des Hinweises auf die Patenterteilung:
**10.04.2002 Patentblatt 2002/15**

(21) Anmeldenummer: **97910283.7**

(22) Anmeldetag: **12.09.1997**

(51) Int Cl.[7]: **C09C 1/00**, C09C 3/12, C09D 7/12, C08K 9/06, A61K 7/00

(86) Internationale Anmeldenummer:
**PCT/EP97/04999**

(87) Internationale Veröffentlichungsnummer:
**WO 98/13426 (02.04.1998 Gazette 1998/13)**

(54) **MODIFIZIERTE PERLGLANZPIGMENTE FÜR WASSERLACKSYSTEME**

MODIFIED NACREOUS LUSTRE PIGMENTS FOR WATER PAINT SYSTEMS

PIGMENTS NACRES MODIFIES POUR SYSTEMES DE VERNIS A L'EAU

(84) Benannte Vertragsstaaten:
**DE ES FI FR GB IT**

(30) Priorität: **27.09.1996 DE 19639783**

(43) Veröffentlichungstag der Anmeldung:
**07.01.1999 Patentblatt 1999/01**

(73) Patentinhaber:
- **MERCK PATENT GmbH**
  **64271 Darmstadt (DE)**
- **Hüls Aktiengesellschaft**
  **45772 Marl (DE)**

(72) Erfinder:
- **GLAUSCH, Ralf**
  **D-64279 Darmstadt (DE)**
- **FORNOFF, Nicole**
  **D-64291 Darmstadt (DE)**
- **DUSCHEK, Joachim**
  **D-64319 Pfungstadt (DE)**
- **STANDKE, Burkhard**
  **D-79540 Lörrach (DE)**
- **EDELMANN, Roland**
  **D-79664 Wehr (DE)**

(56) Entgegenhaltungen:
**EP-A- 0 268 918**
**EP-A- 0 342 533**
**EP-A- 0 632 109**
**EP-A- 0 716 127**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 888 410 B1

Printed by Jouve, 75001 PARIS (FR)

## Beschreibung

Die Erfindung betrifft oberflächenmodifizierte Perlglanzpigmente für Wasserlacksysteme.

Es ist bekannt, daß Titandioxidteilchen, die als Pigmentkomponente in einem Lack enthalten sind, eine oxidative Zersetzung des Polymeren bei Einwirkung von ultravioletten Strahlen und Feuchtigkeit verursachen, die als "Weißwerden" bezeichnet wird. Um diese Wirkung des Titandioxids zu unterdrücken, ist vorgeschlagen worden, Titandioxid mit Chrom-, Silicium-, Aluminium-, Zink-, Phosphor- oder Zirkoniumverbindungen zu beschichten oder zu dopen.

Die EP-A-0 268 918 beschreibt ein wetterbeständiges Perlglanzpigment mit einer hydratisierten Zirkoniumoxidbeschichtung auf dem Titandioxid-Basispigment, die durch Hydrolyse eines Zirkoniumsalzes in Gegenwart eines Hypophosphits erhalten wird.

Die EP-A-0 342 533 beschreibt eine wetterbeständiges Perlglanzpigment mit einer Deckschicht auf dem Titandioxid-Basispigment, die aus hydratisiertem Zirkoniumoxid, das durch Hydrolyse in Gegenwart eines Hypophosphits erhalten wurde und einem hydratisiertem Metalloxid besteht. Das Metalloxid kann dabei Kobalt-, Mangan- oder Ceroxid sein.

Die modifizierten Perlglanzpigmente besitzen eine ausreichend Dispergierbarkeit und Wetterbeständigkeit in nichtwäßrigen Lacksystemen. Für eine Verwendung in wasserverdünnbaren Lacksystemen sind sie aber nicht geeignet, da sie die Bildung mikrofeiner Bläschen im Lackfilm verursachen, die die Lichtstreuung wesentlich erhöhen und damit Glanz und Farbe nachteilig beeinflussen. Außerdem wird die Abbildungsklarheit (DOI) stark vermindert und das Regenerationsvermögen der Lackschicht verschlechtert.

US 5 472 491 beschreibt ein Perlglanzpigment auf der Basis eines mit Metalloxiden beschichteten, plättchenförmigen Substrates und einer auf der Metalloxidschicht befindlichen Deckschicht, die aus Siliciumdioxid, mindestens einem weiteren Metallhydroxid bzw. Metallhydrat der Elemente Cer, Aluminium oder Zirkon und mindestens einem organischen Kupplungsreagenz besteht. Dieses Kupplungsreagenz kann ein Zirkonaluminat, ein Metallsäureester oder ein organofunktionelles Silan sein. Die Kupplungsreagenzien müssen vor der Bindung an die Pigmentoberfläche hydrolysiert werden. Bei Verwendung mehrerer Verbindungen treten Probleme infolge unterschiedlicher Hydrolysegeschwindigkeiten auf. Außerdem können maximal 60 % der zugesetzten Kupplungsreagenzien an die Pigmentoberfläche gebunden werden, wodurch der Materialeinsatz entsprechend erhöht werden muß. Die nicht gekoppelten Anteile verbleiben im Reaktionsmedium und verschlechtern die Filtrierarbeit des Pigments. Für die Bindung des Pigmentes an das Lackbindemittel ist es aber erforderlich, daß unterschiedliche funktionelle Gruppen auf der Pigmentoberfläche vorhanden sind.

Es ist deshalb Aufgabe der vorliegenden Erfindung, ein Perlglanzpigment mit einer Nachbeschichtung zur Verfügung zu stellen, die Kupplungsreagenzien enthält, die hydrophobe und chemisch reaktive Gruppen in einem Molekeil besitzen.

Diese Aufgabe wird gemäß der vorliegenden Erfindung gelöst durch ein Perlglanzpigment auf der Basis eines mit Metalloxiden beschichteten, plättchenförmigen Substrates und einer auf der Metalloxidschicht befindlichen Deckschicht, wobei die Deckschicht aus mindestens zwei Oxiden und/oder gemischten Oxiden und/oder Mischoxiden aus der Gruppe Siliciumdioxid, Aluminiumoxid, Ceroxid, Titandioxid und Zirkoniumoxid und einem wasserbasierenden oligomeren Silansystem besteht.

Das wasserbasierende, oligomere Silansystem ist aus EP 0 675 128, EP 0 716 127 und EP 0 716 128 bekannt. Es wird durch

- Mischen wasserlöslicher Aminoalkylalkoxysilane der allgemeinen Formel I

$$R - Si (R^1)_y(OR^{1*})_{3-y} \qquad (I),$$

vorzugsweise Aminopropyltriethoxysilan, Aminopropylmethyldiethoxysilan, Aminopropyltrimethoxysilan oder Aminopropylmethyldimethoxysilan,
mit nicht wasserlöslichen Alkyltrialkoxysilanen der allgemeinen Formel IIa

$$R^2 - Si (OR^{1**})_3 \qquad (IIa),$$

vorzugsweise Propyltrimethoxysilan, Propyltriethoxysilan, Methyltriethoxysilan, Methyltrimethoxysilan, Ethyltrimethoxysilan, Ethyltriethoxysilan, Isobutyltrimethoxysilan oder Isobutyltriethoxysilan,
und/oder nicht wasserlöslichen Dialkyldialkoxysilanen der allgemeinen Formel III

$$AA' - Si(OR^{1***})_2 \quad (III),$$

vorzugsweise Dimethyldimethoxysilan, Dimethyldiethoxysilan, Methylpropyldimethoxysilan oder Methylpropyldiethoxysilan,
und/oder Mischungen aus nicht wasserlöslichen Alkyltrialkoxysilanen und Dialkyldialkoxysilanen der allgemeinen Formeln II und III,

wobei R eine aminofunktionelle organische Gruppe ist,

$R^1$, $R^{1*}$, $R^{1**}$ und $R^{1***}$ einen Methyl- oder Ethyl-Rest,

$R^2$ einen linearen oder cyclischen oder verzweigten Alkyl-Rest mit 1 bis 8 C-Atomen,

A einen unverzweigten oder verzweigten Alkyl-Rest mit 1 bis 3 C-Atomen und

A' einen unverzweigten oder verzweigten Alkyl-Rest mit 1 bis 3 C-Atomen darstellen und

$0 \leq y < \leq 1$ ist,

- Versetzen des Gemisches mit Wasser und
- Einstellen des pH-Wertes der Reaktionsmischung auf einen Wert zwischen 1 und 8 und
- Entfernen des bereits vorhandenen und/oder bei der Umsetzung entstandenen Alkohols hergestellt.

Das oligomere Silansystem ist weiterhin herstellbar durch

- Mischen von Q Molen wasserlöslicher Aminoalkylalkoxysilane der allgemeinen Formel I

$$R - Si(R^1)_y (OR^{1*})_{3-y} \quad (I),$$

vorzugsweise Aminopropyltriethoxysilan, Aminopropylmethyldiethoxysilan, Aminopropyltrimethoxysilan oder Aminopropylmethyldimethoxysilan,
mit M Molen nicht wasserlöslicher Alkylalkoxysilane der allgemeinen Formel IIb

$$R^3 - Si(OR^{1**})_3 \quad (IIb),$$

wobei R eine aminofunktionelle organische Gruppe ist,

$R^1$, $R^{1*}$ und $R^{1**}$ einen Methyl- oder Ethyl-Rest und

$R^3$ einen linearen-oder cyclischen oder verzweigten Alkylrest mit 1 bis 6 C-Atomen oder eine Ureido-Alkylgruppe der allgemeinen Formel IV

$$NH_2 - CO - NH - (CH_2)_b -, \text{ mit } 1 \leq b \leq 6, \quad (IV)$$

vorzugsweise b = 3,
darstellt und
$0 \leq y \leq 1$ ist,

- in dem molaren Verhältnis $0 < M/Q \leq 2$,
- Versetzen des Gemisches mit Wasser,

- Einstellen des pH-Wertes der Reaktionsmischung auf einen Wert zwischen 1 und 8 und
- Entfernen des bereits vorhandenen und/oder bei der Umsetzung entstandenen Alkohols.

Ferner ist das oligomere Silansystem erhältlich durch

- Mischen wasserlöslicher Organosilane der allgemeinen Formel V

$$H_2N(CH_2)_f(NH)_g(CH_2)_i - Si(CH_3)_h(OR^0)_{3-h} \quad (V),$$

worin $0 \le f \le 6$, g = 0 falls f = 0, g = 1 falls f > 1,$0 \le i \le 6.0 \le h \le 1$ und $R^0$ eine Methyl-, Ethyl-, Propyl- oder Isopropyl-Gruppe sind,

vorzugsweise Aminopropyltriethoxysilan, mit wasserlöslichen, jedoch in wässrigem Medium nicht stabilen Organosilanen der allgemeine Formel VI

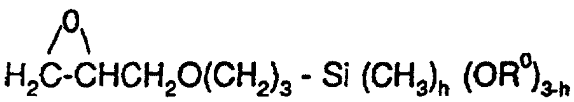

(VI),

worin $0 \le h \le 1$ ist und $R^0$ einen Methyl-, Ethyl-, Propyl- oder Isopropyl-Rest darstellt,
vorzugsweise Glycidyloxypropyltrimethoxysilan,
und/oder der allgemeinen Formel VII

$$H_2C{=}CR'{-}COO(CH_2)_3 - Si(CH_3)_h(OR^0)_{3-h} \quad (VII),$$

worin $0 \le h \le 1$ ist, $R^0$ einen Methyl-, Ethyl-, Propyl- oder Isopropyl-Rest und R' einen Methyl- oder Wasserstoff-Rest darstellen,
vorzugsweise Methacryloxypropyltrimethoxysilan,
und ein nicht wasserlösliches Organosilan der allgemeinen Formel VIII

$$R''{-}Si(CH_3)_h(OR^0)_{3-h} \quad (VIII),$$

worin $0 \le h \le 1$ ist, $R^0$ einen Methyl-, Ethyl-, Propyl- oder Isopropyl-Rest und R'' einen linearen, verzweigten oder cyclischen Kohlenwasserstoff-Rest mit 1 bis 8 C-Atomen darstellen,
vorzugsweise Propyltrimethoxysilan,

- in dem molaren Verhältnis M = a / (b + c + d),
  wobei a die Summe der Molzahlen der Organosilane gemäß Formel V, b die Summe der Molzahlen der Organosilane gemäß Formel VI sowie c die Summe der Molzahlen der Organosilane
  gemäß Formel VII und d die Summe der Molzahlen der Organosilane gemäß Formel VIII sind, mit $0 \le M \le 3$, insbesondere für M gleich 0 mit a gleich 0 und/oder c gleich d gleich 0 und $b \ge 1$ sowie vorzugsweise $0,5 \le M \le 3$,
- Versetzen des Gemisches mit einem Wasser/Säure-Gemisch,
- Einstellen des pH-Wertes der Reaktionsmischung auf einen Wert zwischen 1 und 8 und
- Entfernen des bereits vorhandenen und/oder bei der Umsetzung entstandenen Alkohols.

Bevorzugt gibt man während der destillativen Abtrennung des Alkohols Wasser in dem Maße zu, wie Alkohol bzw. Alkohol/Wasser-Gemisch aus dem Reaktionsmedium entfernt wird. Zum Einstellen des pH-Wertes sind einbasige Säuren besonders geeignet. So hergestellte Produkte setzen auch bei Verdünnen mit Wasser keine weiteren Alkohole

durch Hydrolyse frei und weisen einen Flammpunkt von deutlich mehr als 70 °C auf.

Ein oligomeres Silansystem, das auf die oben beschriebene Art und Weise hergestellt werden kann, ist beispielsweise VPHS 2927, ein oligomeres Silansystem der Hüls AG, das die folgende Zusammensetzung und Näherungsstruktur besitzt:

$$[HO[Si(A)(OH)O]_3[Si(B)(OH)O_1[Si(C)(OH)O]_1[Si(D)(OH)O]_1H \cdot (HX)_3]_n$$

wobei

A = 3-Aminopropyl-
B = Glycidyloxypropyl-
C = Methacryloxypropyl-
D = Propyl-

$$n \geq 1$$

Weiterhin wird die Aufgabe gemäß der vorliegenden Erfindung durch ein Verfahren zur Herstellung eines Perlglanzpigmentes auf der Basis eines mit Metalloxiden beschichteten, plättchenförmigen Substrates auf einer auf der Metalloxidschicht befindlichen Deckschicht gelöst, indem man das mit Metalloxiden beschichtete Substrat in Wasser suspendiert, in einer ersten Stufe durch Zusatz geeigneter Zubereitungen, beispielsweise wäßriger Salzlösungen oder organischer Metallverbindungen von mindestens 2 Elementen aus der Gruppe Silicium, Aluminium, Cer und Zirkonium die entsprechenden Oxidhydrate entweder gleichzeitig oder nacheinander bei einer Temperatur von 30 bis 100 °C, vorzugsweise 40 bis 75 °C, ausfällt, wobei die Oxidhydrate von Silicium und Aluminium bei einem pH-Wert von 5 bis 9 und die Oxidhydrate von Cer und Zirkonium bei einem pH-Wert von 3 bis 7 auf das Substrat aufgefällt werden und durch gleichzeitige Zugabe von Säure oder Lauge oder einem geeignetem Puffersystem der pH-Wert konstant gehalten wird und in einer zweiten Stufe bei einem pH-Wert von 3 bis 8 ein oligomeres Silansystem zusetzt, das auf der Pigmentoberfläche gebunden wird und anschließend das Pigment abtrennt, mit vollentsalztem Wasser wäscht und bei 80° bis 160 °C, vorzugsweise 120° bis 160 °C, trocknet.

Gegenstand der Erfindung ist außerdem die Verwendung der erfindungsgemäßen Pigmente zur Pigmentierung von Lacken, Druckfarben, Kunststoffen und Kosmetika. Hierfür können sie als Mischungen mit handelsüblichen Pigmenten, beispielsweise anorganischen und organischen Absorptionspigmenten, Metalleffektpigmenten und LCP-Pigmenten, eingesetzt werden.

Als Substrat werden Pigmente verwendet, die aus einem plättchenförmigen Material, beispielsweise Glimmer, Kaolin oder Glas und einer oder mehrerer darauf abgeschiedener Metalloxidschichten bestehen. die Metalloxidschicht kann beispielsweise aus Titandioxid, Eisen(III)oxid, Chromoxid, Zirkondioxid, Zinndioxid, Zinkoxid oder Gemischen derselben bestehen. Derartige Pigmente sind im Handel unter der Bezeichnung Iriodin® (Hersteller: Merck KGaA, Darmstadt) erhältlich.

Der Anteil der Deckschicht am gesamten Pigment beträgt 1 bis 20 Gew.-%, bevorzugt 4 bis 10 Gew.-%. Davon entfallen 2 bis 8 Gew.-% auf die Metalloxide und 2 bis 12 Gew.-% auf das wasserbasierende oligomere Silansystem.

Die verwendeten Silane bzw. wasserbasierenden oligomeren Silansysteme sind in den Europäischen Patentanmeldungen Nr. 0 675 128, 0 716 127 und 0 716 128 näher beschrieben.

Die erhaltenen wasserbasierenden oligomeren Silansysteme (organopolysiloxan-haltige Zusammensetzungen auf Wasserbasis) sind im wesentlichen frei von organischen Lösemitteln und besitzen einen Flammpunkt von mehr als 70 °C. Da durch die Mischung mit Wasser die Alkoxygruppen bereits im wesentlichen hydrolysiert wurden, werden beim Verdünnen mit Wasser weniger als 5 Gew.-% Alkohole (Methanol, Ethanol) durch Hydrolyse freigesetzt.

Es resultieren beispielsweise Verbindungen mit folgender Näherungsstruktur (Formel IX)

Formel IX:

$$[HO[Si(A)(OH)_Z(CH_3)_{1-Z}O]_a[Si(B)(OH)_Y(CH_3)_{1-Y}O]_b[Si(C)(OH)_W(CH_3)_{1-W}O]_c[Si(D)(OH)_V(CH_3)_{1-V}O]_d[H \cdot (HX)_e,$$

wobei

A = Aminoalkylrest abgeleitet aus der allgemeinen Formel V,
B = Glycidetheralkylrest abgeleitet der allgemeinen Formel VI,
C = Acryloxyalkyl- oder Methacryloxyalkylrest der allgemeinen Formel VII,
D = Alkylrest der allgemeinen Formel VIII,
HX = einbasige Säure, wobei X = anorganischer oder organischer Säurerest, wie z.B. Chlorid, Nitrat, Formiat, Acetat,

v gleich 0 oder 1 und w gleich 0 oder 1 und y gleich 0 oder 1 und z gleich 0 oder 1 und $a + b + c + d \geq 4$ und $a \leq e \leq 2a$ sind, mit $0 \leq a / (b + c + d) \leq 3$, insbesondere für $a / (b + c + d)$ gleich 0 mit a = 0 und/oder c gleich d gleich 0 und $b \geq 1$ sowie für $0{,}5 \leq a / (b + c + d) \leq 3$.

Aufgabe der siliciumfunktionellen Hydroxylgruppen ist es, chemische Bindungen zu den Hydroxylgruppen der Pigmentoberfläche auszubilden. Dadurch wird ein stabiler Verbund zwischen Silan und Pigmentoberfläche aufgebaut.

Die organofunktionellen Gruppen des oligomeren Silans haben die Aufgabe, Bindungen zum Polymer des Wasserlackes herzustellen. Da die Oligomeren mit mehreren, sich voneinander unterscheidenden funktionellen Gruppen ausgerüstet werden können, kann das Pigment in unterschiedlichen Wasserlacksystemen verwendet werden. Eine Ausrüstung mit Methacryl- und Aminogruppen bedeutet zum Beispiel, daß das Pigment für Wasserlacksysteme mit Polyester als Polymer und für Wasserlacksysteme mit Polyurethan als Polymer einsetzbar ist.

In einer ersten Ausführungsform werden erfindungsgemäßen Pigmente hergestellt, indem auf die Metalloxidschicht des Substrates (Basispigment) in einer ersten Stufe 0,1 bis 10 Gew.-%, vorzugsweise 0,5 bis 3 Gew.-%, Siliciumdioxid abgeschieden werden. Dazu wird zu einer wäßrigen 1 bis 35%ige, vorzugsweise 5 bis 20%ige, Suspension des Basispigmentes im pH-Bereich von 6 bis 9 eine verdünnte Natriumsilicatlösung zudosiert. Der pH-Wert wird durch Zusatz von Salzsäure konstant gehalten, wobei alle angegebenen pH-Werte mit Hilfe einer kalibrierten Elektrode bestimmt werden.

In einer zweiten Stufe werden nach Einstellung des pH-Wertes auf einen Bereich von 3 bis 5, wäßrige Salzlösungen oder feste Salze der Metalle Cer, Aluminium oder Zirkon oder deren Gemische in einer Konzentration von 1 bis 4 Gew.-%, bezogen auf Oxid, in Gegenwart eines löslichen Sulfates zu einer wäßrigen Suspension des in Stufe 1 beschichteten Substrats zugegeben, der pH-Wert im Bereich 3 bis 5 konstant gehalten und die Suspension auf 30 bis 100 °C, vorzugsweise 40 bis 75 °C, unter Rühren für 5 min bis 4 Stunden, vorzugsweise 30 min bis 2 Stunden, erhitzt.

Unter diesen Bedingungen wird nur ein Teil, maximal 50 % der Metallionen als Hydroxide oder Oxidhydrate ausgefällt.

In einer dritten Stufe werden dann 1 bis 20 Gew.-%, vorzugsweise 2 bis 12 Gew.-%, oligomeres Silansystem bezogen auf das eingesetzte Pigment zugegeben, die Suspension 5 min bis 4 Stunden, vorzugsweise 30 min bis 2 Stunden, bei einem pH-Wert von 3 bis 5 gerührt und dann innerhalb von 5 min bis 4 Stunden, vorzugsweise 30 min bis 2 Stunden, der pH-Wert auf einen Bereich von 5 bis 8 eingestellt, wobei die Suspension bei einer Temperatur von 30 bis 100 °C, vorzugsweise 40 bis 75 °C gehalten wird.

Unter diesen Bedingungen erfolgt eine vollständige Mischfällung der restlichen Metallionen als Hydroxide oder Oxidhydrate zusammen mit dem oligomeren Silansystem.

Anschließend wird das Pigment abgetrennt, salzfrei gewaschen und bei 80 bis 180 °C, vorzugsweise 120 bis 160 °C, getrocknet.

In einer zweiten Ausführungsform werden die erfindungsgemäßen Pigmente hergestellt, indem auf die Metalloxidschicht des Substrates in einer ersten Stufe 0,1 bis 10 Gew.-%, vorzugsweise 0,5 bis 3 Gew.-% (berechnet auf $Al_2O_3$) Aluminiumoxidhydrat abgeschieden werden. Dazu wird zu einer wäßrigen, 1 bis 35%igen, vorzugsweise 5 bis 20%igen Suspension des Basispigmentes im pH-Bereich von 7 bis 9 und bei einer Temperatur von 40 bis 80 °C eine verdünnte Aluminiumchloridlösung über einen Zeitraum von 2 Stunden zudosiert. Der pH-Wert wird durch gleichzeitige Zugabe von Natronlauge konstant gehalten.

Unter den gleichen Bedingungen wie für die Fällung des Aluminiumoxidhydrates wird anschließend in einer zweiten Stufe durch Zugabe einer verdünnten Natriumsilikatlösung Siliciumdioxidhydrat in einer Menge von 0,1 bis 10 Gew.-%, vorzugsweise 0,5 bis 3 Gew.-%, bezogen auf das Basispigment auf die Aluminiumoxidhydratschicht aufgefällt. Durch gleichzeitige Zugabe von Salzsäure wird der pH-Wert im Bereich von 7 bis 9 konstant gehalten.

In einer dritten Stufe wird dann eine 20 bis 60%ige wäßrige Lösung eines oligomeren Silansystems bei 40 bis 80 °C über einen Zeitraum von 10 Min. bis 2 Stunden, vorzugsweise 30 bis 90 Min., der Pigmentsuspension zudosiert. Die zudosierte Menge beträgt 2 bis 12 Gew.-% bezogen auf das Basispigment. Anschließend wird das Pigment aus der Reäktionslösung abgetrennt, salzfrei gewaschen und bei 80 bis 180 °C, vorzugsweise 120 bis 160 °C, getrocknet.

In einer weiteren Ausführungsform kann die Auffällung von Aluminiumoxidhydrat und Siiciumoxidhydrat auf das Basispigment auch durch Hydrolyse eines Silicium/Aluminium-Esters der allgemeinen Formel X

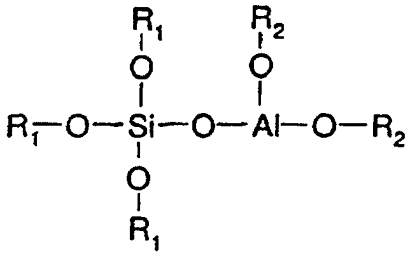

(X)

erfolgen, worin $R_1$ und $R_2$ gleich oder verschieden sein können und eine Alkylgruppe mit 1 bis 8 Kohlenstoffatomen darstellen. Vorzugsweise wird eine Verbindung eingesetzt, worin $R_1$ eine Ethylgruppe und $R_2$ eine sekundäre Butylgruppe ist. Diese Verbindung wird unter der Bezeichnung DYNASIL® Si-Al von der Hüls Aktiengesellschaft, Marl vertrieben.

Der in Ethanol gelöste Silicium/Aluminium-Ester wird zu der auf 30 bis 50 °C erwärmten Suspension des Basispigmentes bei einem pH-Wert von 4 bis 9, bevorzugt 5,5-7,5, über einen Zeitraum von 10 Min. bis 2 Stunden, vorzugsweise 30 bis 90 Min., zudosiert. Anschließend wird dann dem Reaktionsgemisch das oligomere Silansystem in der beschriebenen Weise zugegeben.

In einer zusätzlichen Ausführungsform können an Stelle des oligomeren Silansystems oder zusätzlich zum oligomeren Silansystem ein oder mehrere monomere Silane eingesetzt werden. Diese monomeren Silane können sein ein Alkyltrialkoxysilan (insbesondere Methyltrimethoxysilan, Methyltriethoxysilan, Ethyltrimethoxysilan, Ethyltriethoxysilan, Propyltrimethoxysilan, Propyltriethoxysilan, Isobutyltrimethoxysilan, Isobutyltriethoxysilan, Octyltrimethoxysilan, Octyltriethoxysilan) oder
ein Dialkyldialkoxysilan (insbesondere Dimethyldimethoxysilan, Dimethyldiethoxysilan, Propylmethyldimethoxysilan, Propylmethyldiethoxysilan) oder
ein Glycidyloxyalkylalkoxysilan (insbesondere Glycidyloxypropyltrimethoxysilan, Glycidyloxypropyltriethoxysilan, Glycidyloxypropylmethyldimethoxysilan, Glycidyloxypropylmethyldiethoxysilan) oder
ein Methacryloxyalkylalkoxysilan (insbesondere Methacryloxypropyltrimethoxysilan, Methacryloxypropyltriethoxysilan, Methacryloxypropylmethyldimethoxysilan, Methacryloxypropylmethyldiethoxysilan) oder
ein Vinylalkoxysilan (insbesondere Vinyltrimethoxysilan, Vinyltriethoxysilan, Vinyltrimethoxyethoxysilan, Vinylmethyldimethoxysilan, Vinylmethyldiethoxysilan) oder ein Gemisch derselben.

In einer dritten Ausführungsform werden die erfindungsgemäßen Pigmente hergestellt, indem auf die Metalloxidschicht des Substrates (Basispigment) in einer ersten Stufe 0,1 bis 5 Gew.-%, vorzugsweise 0,5 bis 3 Gew.-% Ceroxidhydrat abgeschieden werden. Dazu wird zu einer wäßrigen 1 bis 35%igen, vorzugsweise 5 bis 20%igen Suspension des Basispigmentes, die auf 40 bis 80 °C aufgeheizt wird, im pH-Bereich von 1 bis 4 eine verdünnte Cerchloridlösung innerhalb von 20 Min. zudosiert, wobei durch Zugabe von Natronlauge der pH-Wert konstant gehalten wird.

In einer zweiten Stufe wird unter den gleichen Reaktionsbedingungen ein Gemisch aus Zirkoniumoxidchlorid, Natriumhypophosphit und Salzsäure über einen Zeitraum von 10 Min. bis 2 Stunden, vorzugsweise 30 bis 90 Min., zudosiert und Zirkoniumoxidhydrat auf die Ceroxydhydratschicht aufgefällt. Die abgeschiedene Menge an Zirkoniumoxidhydrat beträgt etwa 0,1 bis 10 Gew.-%, vorzugsweise 0,1 bis 5 Gew.-% $ZrO_2$ bezogen auf das Basispigment.

Da unter den angegebenen Bedingungen die Oxidhydrate von Zirkonium und Cer nicht vollständig gefällt werden, wird in einer dritten Stufe durch Zugabe von Natronlauge innerhalb von 30 Min. der pH-Wert auf 9 angehoben und die Fällung der Oxidhydrate vervollständigt.

Nach 60 Min. Nachrühren wird bei einem pH-Wert von 7 bis 9 eine 20 bis 60%ige wäßrige Lösung eines oligomeren Silansystems bei 40 bis 80 °C über einen Zeitraum von einer Stunde der Pigmentsuspension zudosiert. Anschließend wird das Pigment aus der Reaktionslösung abgetrennt, salzfrei gewaschen und bei 80 bis 180 °C, vorzugsweise 120 bis 160 °C, getrocknet.

Die so erhaltenen farbneutralen Pigmente zeigen eine sehr gute Wetterbeständigkeit, was durch die nachfolgend aufgeführten Testergebnisse belegt wird. Das Pigment ist einerseits gut rieselfähig und besitzt andererseits eine sehr gute Eignung bezüglich Dispergierbarkeit, Stabilität, Coloristik, Microblasenbildung, Quellung und Glanz für wasserverdünnbare Lacksysteme, insbesondere Autolacksysteme.

Durch die Modifizierung der Pigmentoberfläche mit unterschiedlichen funktionellen Gruppen kann das Pigment für die verschiedenen Wasserlacksysteme eingesetzt werden, ohne daß eine spezielle Anpassung notwendig ist.

Nach den folgenden 2 Prüfmethoden wurden die erfindungsgemäßen Pigmente getestet:

**Wasser-Immersionstest**

Die Pigmentproben wurden in ein konventionelles Lacksystem eingearbeitet und die Prüfproben per Spritzapplikation hergestellt. Die Prüfung erfolgte im Einschichtsystem nach 16 Stunden bei 66 °C und nach 20 Stunden bei 80 °C. Dabei werden die Prüfproben zur Hälfte in destilliertes Wasser eingetaucht. Die visuelle Beurteilung der Graufärbung nach Bewitterung erfolgte nach ISO 105 Part A 02 (entspricht DIN 54 001) 24 Stunden nach Belastungsende. Die Beurteilungsskala reicht von 5 (sehr gut) bis 1 (sehr schlecht).

**Kondenswasserprüfung**

Die Pigmentproben wurden in ein Wasserlacksystem eingearbeitet und die Prüfproben per Spritzapplikation hergestellt.

Die Prüfung erfolgte nach DIN 50 017 (Kondenswasser-Konstantklimate) 10 Min. bis eine Stunde nach Belastungsende.

Die Beurteilung des Blasengrades erfolgte visuell nach DIN 53 209. "m" bedeutet Häufigkeit der Blasen je Flächeneinheit und "g" bedeutet Größe der Blasen. Die Bewertungsskala reicht von 0 (sehr gut) bis 5 (sehr schlecht), demnach umgekehrt zu den beiden vorangehenden Prüfmethoden.

Die Beurteilung des Quellungsvorganges erfolgte visuell in Anlehnung an DIN 53 230, Tabelle 2. In der relativen Bewertungsskala bedeutet die Kennzahl 0: "nicht verändert" und die Kennzahl 5: "sehr stark verändert".

Tabelle 1 enthält die Testergebnisse für die nach den Beispielen 1 bis 8 hergestellten Pigmente gemäß der vorliegenden Erfindung nach den oben beschriebenen Prüfmethoden.

Die am Ende aufgeführte Nullprobe bestand aus den reinen Wasserlacksystemen ohne Pigment. Die Nullprobe zeigt, daß auch die reinen Wasserlacksysteme geringe Quellungen aufweisen.

Die verwendeten Basispigmente wurden vor dem Nachbeschichten mit Ausnahme von Beispiel 8 aufgereinigt. Dazu wurden sie in Wasser zu einer 10%igen Suspension eingetragen, wobei das Wasser auf einen pH-Wert von 10 bis 11 eingestellt worden war. Die Suspension wurde über Nacht stehen gelassen und die überstehende trübe Lösung abdekantiert.

## Tabelle 1

| Beispiel Nr. | verwendetes Basispigment | | | eingesetztes Hydrosil VPHS 2909* | Filtrationszeit (min) | Ausbeute (%) bezogen auf Basispigm. | Elementaranalyse | | | Wassereintauchtest (→ konv. Lacksystem) | | Kondenswassertest (→ Wasserlacksystem) | | |
|---|---|---|---|---|---|---|---|---|---|---|---|---|---|---|
| | Typ | Aufreinigung | chem. Aufbau | | | | C (%) | H (%) | N (%) | 16 h 66 °C | 20 h 80 °C | 1 | 2 | 3 |
| 1 | Ir. 103 | JA | $TiO_2$ geringe Schichtdicke | 3 % | 120 | 102 | 1,0 | 0,2 | 0,1 | 5 | 5-4 | 1,0 | | |
| 2 | Ir. 215 | JA | $TiO_2$ mittlere Schichtdicke | 3 % | 85 | 102 | 0,7 | 0,2 | nicht nachweisbar | 5-4 | 4 | 1,0 | | |
| 3 | Ir. 235 | JA | $TiO_2$ hohe Schichtdicke | 3 % | 75 | 101,8 | 0,6 | 0,2 | nicht nachweisbar | 5 | 5-4 | 1,0 | | |
| 4 | Ir. 7235 | JA | $TiO_2$ sehr hohe Schichtdicke | 3 % | 40 | 102,2 | 0,6 | 0,2 | nicht nachweisbar | 5 | 5-4 | 1,0 | | |
| 5 | Ir. 444 | JA | $TiO_2$ + $Cr_2O_3$ Mischoxide | 3 % | 50 | 101,9 | 0,6 | 0,2 | nicht nachweisbar | 5 | 5-4 | 1,0 | | |
| 6 | Ir. 303 | JA | $TiO_2$ + $Fe_2O_3$ Mischoxide | 3 % | 95 | 102,7 | 0,6 | 0,2 | nicht nachweisbar | 5 | 5-4 | 1,0 | | |
| 7 | Ir. 504 | JA | $Fe_2O_3$ mittlere Schichtdicke | 3 % | 160 | 102,9 | 0,6 | 0,2 | nicht nachweisbar | 5 | 4-5 | 1,8 | | |
| 8 | Ir. 504 | NEIN | $Fe_2O_3$ mittlere Schichtdicke | 3 % | 135 | 102,9 | 0,6 | 0,2 | nicht nachweisbar | 5-4 | 4-5 | 1,8 | | |
| Nullprobe | Wasserlack | | | | | | | | | | | 1,3 | | |

* Hydrosil VPHS 2909: Bezeichnung des erfindungsgemäß eingesetzten oligomeren Silansystems gemäß Beispiel 5 der EP 0 716 127

Tabelle 2 enthält zum Vergleich die Testergebnisse für die in den Vergleichsbeispielen 1 bis 6 verwendeten konventionellen Pigmente. Als Basispigment wurde Iriodin® 9225 Rutil Perlblau, ein mit Titandioxid beschichtetes Glimmerpigment verwendet. In den Vergleichsbeispielen 1 bis 4 war es mit einer Nachbeschichtung aus Siliciumdioxid, Aluminiumoxid, Ceroxid und einem oder mehreren Silanen gemäß US 5 472 491 beschichtet. Im Vergleichsbeispiel 5 war es mit Zirkonoxidhydrat, Ceroxidhydrat und einem Epoxysilan gemäß EP 0 342 533 beschichtet. Im Vergleichsbeispiel 6 was es mit einer zusätzlichen Beschichtung aus Chromund Manganverbindungen und zwei Silanen mit Methacrylgruppen bzw. sekundären Aminogruppen versehen.

Ein Vergleich der Testergebnisse nach dem Kondenswassertest in beiden Tabellen zeigt, daß die Pigmente gemäß der vorliegenden Erfindung in Wasserlacksystemen deutlich stabiler sind als die in Tabeller 2 enthaltenen konventionellen Pigmente. Eine Ausnahme bilden lediglich die in den Beispielen 7 und 8 verwendeten Eisenoxidpigmente. Ihre Stabilität entspricht etwa den Pigmenten gemäß US 5 472 491. Sie haben aber den Vorteil, daß sie etwa 70 % weniger Silane enthalten und in verschiedenen Wasserlacksystemen einsetzbar sind.

## Tabelle 2

| Vergleichsbeispiel Nr. | Basispigment | Stand der Technik | verwendete Silane bzw. Zr/Al-Kuppler | Funktionalität | Filtrationszeit (min) | Ausbeute (%) bezogen auf Basis-Pigment | Beurteilung des getrockneten nachbeschichteten Pigmentes | Elementaranalyse C (%) | Elementaranalyse H (%) | Elementaranalyse N (%) | Wassereintauchtest (→ konv. Lacksystem) 16 h 66 °C | Wassereintauchtest (→ konv. Lacksystem) 20 h 80 °C | Kondenswassertest (→ Wasser-Lacksystem) 1 | Kondenswassertest (→ Wasser-Lacksystem) 3 |
|---|---|---|---|---|---|---|---|---|---|---|---|---|---|---|
| 1 | Ir. 225 | US 5 472 491 Beispiel 1 | 3 % AMMO | prim. amino | 90 | 101,7 | weich leicht zu sieben | 0,2 | 0,1 | nicht nachweisbar | 5 | 4 | 2,0 | |
| 2 | Ir. 225 | US 5 472 491 Beispiel 2 | 3 % AMMO | prim. amino | 95 | 102,8 | etwas härtere Brocken leicht zu sieben | 0,5 | 0,2 | nicht nachweisbar | 4-5 | 3-4 | 1,9 | |
| | | | + 5 % GLYMO | + epoxy | | | | | | | | | | |
| | | | + 3 % CPG | + carboxy | | | | | | | | | | |
| 3 | Ir. 225 | US 5 472 491 Beispiel 2 | 3 % AMMO | prim. amino | 85 | 104,4 | härtere Brocken leicht zu sieben | 1,4 | 0,3 | 0,2 | 5 | 4-5 | 1,7 | |
| | | | + 3 % MEMO | + methacryl | | | | | | | | | | |
| | | | + 3 % GLYMO | + epoxy | | | | | | | | | | |
| 4 | Ir. 225 | US 5 472 491 Beispiel 3 | 3 % MEMO | methacryl | 110 | 104,6 | rel. weich leicht zu sieben | 1,0 | 0,3 | nicht nachweisbar | 4-5 | 4-5 | 1,5 | |
| | | | + 5 % GLYMO | + epoxy | | | | | | | | | | |
| | | | + 3 % CPG | + carboxy | | | | | | | | | | |
| 5 | Ir. 225 | EP 0 342 533 | 2 % GLYMO | epoxy | | | weich leicht zu sieben | | | | | | 2,0 | 2,6 |
| 6 | Ir. 225 | EP 0 104 516 | 0,2 % MEMO | methacryl | | | weich leicht zu sieben | | | | | | 2,5 | 2,3 |
| | | | + 0,2 % DAMO | + sek. amino | | | | | | | | | | |
| | Nullprobe | Wasserlack 1 | | | | | | | | | | | 1,1-1,3 | |
| | Nullprobe | Wasserlack 3 | | | | | | | | | | | 1,1 | |

Erläuterungen zu Tabelle 2:

AMMO
3-Aminopropyl-trimethoxysilan
(DYNASYLAN® AMMO)
Hersteller: Hüls AG

GLYMO
3-Glycidyloxypropyl-trimethoxysilan
(DYNASYLAN® GLYMO)
Hersteller: Hüls AG

MEMO
3-Methacryloxypropyl-trimethoxysilan
(DYNASYLAN® MEMO)
Hersteller: Hüls AG

DAMO
N-Aminomethyl-3-aminopropyl-trimethoxysilan
(DYNASYLAN® DAMO)
Hersteller: Hüls AG

CPG (Manchem® C)
Carboxy-Zirkonaluminat-Lösung
Hersteller: Rhône-Poulenc-Chemicals

Die nachfolgenden Beispiele sollen die Erfindung erläutern, ohne sie zu begrenzen.

Beispiel 1

200 g Iriodin® 225 Rutil Perlblau (mit Titandioxid beschichtetes Glimmerpigment der Merck KGaA, Darmstadt) werden in 1,8 l vollentsalztem Wasser suspendiert und unter kräftigem Rühren auf 40 °C aufgeheizt. Mit 5%iger Natronlauge wird der pH-Wert auf 10,5 eingestellt. Der pH-Wert wird generell mit einer kalibrierten Elektrode gemessen.

Anschließend läßt man innerhalb von 30 Min. mit einer Geschwindigkeit von 10 ml/Min. eine Lösung von 10,8 ml Natronwasserglas (185 g $SiO_2$/l) in 300 ml Wasser zur Pigmentsuspension tropfen. Der pH-Wert wird dabei mit 2,5%iger Salzsäure konstant gehalten. Nach beendeter Zugabe wird 15 Min. bei 40 °C nachgerührt und anschließend mit 2,5%iger Salzsäure innerhalb von 20 Min. der pH-Wert auf 7,6 eingestellt. Nun werden 1,35 g Natriumsulfat, 2,30 g Aluminium und 1,10 g Cer(III)chlorid-Heptahydrat als Feststoffe in die Pigmentsuspension zugegeben, wobei der pH-Wert auf 3,4 abfällt.

Anschließend wird innerhalb von 30 Min. auf 75 °C aufgeheizt, wobei der pH-Wert auf 2,9 abfällt. Die Suspension wird für weitere 75 Min. unter rühren bei 75 °C gehalten. Dabei werden die Metalloxidhydrate wenigstens zum Teil, abgeschieden.

Nun werden 15 g VPHS 2927, ein oligomeres Silansystem der Hüls AG, Marl (siehe oben) innerhalb von 10 Min. zugegeben. Der pH-Wert wird durch Zugabe von 2,5%iger Salzsäure bei 2,8 konstant gehalten. Nach beendeter Zugabe wird 120 Min. bei 75 °C nachgerührt. Dann wird durch Zugabe von 2,5%iger Natronlauge innerhalb von 90 Min. der pH-Wert auf 8,5 eingestellt. Dabei werden die Metalloxidhydrate und das oligomere Silansystem vollständig als Mischfällung auf der Pigmentoberfläche abgeschieden.

Anschließend werden noch 60 Min. zur Nachreaktion bei 75 °C weitergerührt, wobei der pH-Wert auf 8,0 abfällt. Nach längerem Stehen wird die überstehende Flüssigkeit abdekantiert, das Pigment mit Wasser angeschlämmt, über eine Filternutsche abgesaugt und 16 Stunden bei 140 °C getrocknet.

Beispiel 2

200 g Iriodin® 225 Rutil Perlblau (mit Titandioxid beschichtetes Glimmerpigment der Merck KGaA, Darmstadt) werden in 1,8l vollentsalztem Wasser suspendiert und unter kräftigem Rühren auf 75 °C aufgeheizt. Mit 2,5%iger Salzsäure wird der pH-Wert auf 8,0 eingestellt.

Anschließend läßt man innerhalb von 120 Min. eine Lösung von 9,5 g $AlCl_3$. 8 $H_2O$ in 100 ml Wasser mit einer Geschwindigkeit von 0,9 ml/Min. zutropfen, dabei wird durch gleichzeitige Zugabe von 2,5%iger Natronlauge der pH-Wert konstant gehalten.

Nach einer Nachrührzeit von 10 Min. wird mit einer Geschwindigkeit von 1,0 ml/Min. innerhalb von 120 Min. mit einer Geschwindigkeit von 1,0 ml/ Min. eine Lösung von 16,2 ml Natronwasserglas (185 g $SiO_2$/l) in 100 ml Wasser zudosiert. Nach beendeter Zugabe wird noch 10 Min. nachgerührt. Während der Fällung wird durch Zugabe von 2,5%iger Schwefelsäure der pH-Wert bei 8,0 gehalten.

Nun werden 15 g VPHS 2927, ein oligomeres Silansystem der Hüls AG, Marl (siehe oben) innerhalb von 10 Min. zugegeben. Der pH-Wert wird durch Zugabe von 2,5%iger Salzsäure bei 2,8 konstant gehalten. Nach beendeter Zugabe wird 120 Min. bei 75 °C nachgerührt. Dann wird durch Zugabe von 2,5%iger Natronlauge innerhalb von 90 Min. der pH-Wert auf 8,5 eingestellt. Dabei wird das oligomere Silansystem vollständig auf der Pigmentoberfiäche gebunden. Anschließend werden noch 60 Min. zur Nachreaktion bei 75 °C weitergerührt, wobei der pH-Wert auf 8,0 abfällt.

Nach längerem Stehen wird die überstehende Flüssigkeit abdekantiert, das Pigment mit Wasser angeschlämmt, über eine Filternutsche abgesaugt und 16 Stunden bei 140 °C getrocknet.

Beispiel 3

200 g Iriodin® 225 Rutil Perlblau (mit Titandioxid beschichtetes Glimmerpigment der Merck KGaA, Darmstadt) werden in 1,8 l vollentsalztem Wasser suspendiert und unter kräftigem Rühren auf 75 °C aufgeheizt. Mit 5%iger Salzsäure wird der pH-Wert auf 2,5 eingestellt.

Anschließend läßt man innerhalb von 20 Min. mit einer Geschwindigkeit von 2,2 ml/Min. eine Lösung von 2,20 g $CeCl_3 \cdot 7\ H_2O$ in 44 ml Wasser zur Pigmentsuspension tropfen, wobei der pH-Wert bei 2,5 konstant bleibt.

Danach wird eine Lösung aus 11,52 g $ZrOCl_2 \cdot 8\ H_2O$, 7,58 g $NaH_2PO_2 \cdot H_2O$ und 11,50 g 37%iger Salzsäure in 668 ml Wasser mit einer Dosiergeschwindigkeit von 11,3 ml/Min. innerhalb von 60 Min. zugegeben und der pH-Wert durch gleichzeitige Zugabe von 5%iger Natronlauge konstant gehalten.

Die Lösung wird hergestellt, indem man bei Raumtemperatur der $ZrOCl_2$-Lösung vorlegt und unter kräftigem Rühren innerhalb von 60 Min. mit einer Geschwindigkeit von 6,3 ml/Min. die $NaH_2PO_2$-Lösung zudosiert. Anschließend wird die konzentrierte Salzsäure innerhalb von 1 Min. zugegeben und nach 10 Min. Rühren eine klare Lösung erhalten. Nach Zugabe des Gemisches wird noch 30 Min. bei 75 °C nachgerührt.

Dann wird mit einer Geschwindigkeit von 2,0 ml/Min. innerhalb von 35 Min. eine 5%ige Natronlauge zudosiert. Der pH-Wert steigt auf 8,5, wodurch eine vollständige Fällung der Oxidhydrate erreicht wird. Anschließend werden 15 g VPHS 2927, ein oligomeres Silansystem der Hüls AG, (siehe oben) als 40%ige wäßrige Lösung mit einer Geschwindigkeit von 1,9 ml/ Min. innerhalb von 60 Min. zugegeben. Durch gleichzeitige Zugabe von NaOH wird der pH-Wert bei 8,5 konstant gehalten. Dabei wird das oligomere Silansystem vollständig auf der Pigmentoberfläche gebunden. Anschließend werden noch 60 Min. zur Nachreaktion bei 75 °C weitergerührt, wobei der pH-Wert auf 8,0 abfällt. Nach längerem Stehen wird die überstehende Flüssigkeit abdekantiert, das Pigment mit Wasser angeschlämmt, über eine Filternutsche abgesaugt und 16 Stunden bei 140 °C getrocknet.

Beispiel 4

200 g Iriodin® 225 Rutil Perlblau (mit Titandioxid beschichtetes Glimmerpigment der Merck KGaA, Darmstadt) werden in 1,8 l vollentsalztem Wasser suspendiert und unter kräftigem Rühren auf 40 °C aufgeheizt. Mit 2,5%iger Salzsäure wird der pH-Wert auf 6,5 eingestellt. Nun wird mit einer Geschwindigkeit von 2,6 ml/Min. eine ethanolische Lösung des Silicium/Aluminium-Esters zudosiert. Unter diesen Bedingungen wird der Ester vollständig hydrolysiert. Das entstehende Aluminiumoxidhydrat und Siliciumdioxidhydrat werden auf das Basispigment aufgefällt.

Anschließend werden dem Reaktionsgemisch 15 g VPHS 2927, ein oligomeres Silansystem der Hüls AG, (siehe oben) zugegeben und gemäß Beispiel 3 weitergearbeitet.

Beispiel 5

200 g Iriodin® 225 Rutil Perlblau (mit Titandioxid beschichtetes Glimmerpigment der Merck KGaA, Darmstadt) werden in 1,8 l vollentsalztem Wasser suspendiert und gemäß Beispiel 4 weitergearbeitet. Anstelle des oligomeren Silansystems wird aber Propyltrimethoxysilan, ein monomeres Silan (Hersteller: Hüls AG) unter den gleichen Bedingungen zudosiert. Das Pigment wird dann gemäß Beispiel 3 aufgearbeitet.

## Patentansprüche

**1.** Modifizierte Perlglanzpigmente für Wasserlacksysteme auf der Basis eines mit Metalloxiden beschichteten, plättchenförmigen Substrates und einer auf der obersten Metalloxidschicht befindlichen Deckschicht, wobei die Deckschicht aus mindestens zwei Oxiden und/oder gemischten Oxiden und/oder Mischoxiden aus der Gruppe Siliciumdioxid, Aluminiumoxid, Ceroxid, Titanoxid und Zirkoniumoxid und einem wasserbasierenden oligomeren Silansystem besteht, das im wesentlichen frei von organischen Lösungsmitteln ist, einen Flammpunkt von mehr als 70° besitzt und bei Verdünnen mit Wasser weniger als 5 Gew.-%. Alkohole durch Hydrolyse freisetzt,
erhältlich durch

- Mischen wasserlöslicher Aminoalkylalkoxysilane der allgemeinen Formel I

$$R - Si\,(R^1)_y\,(OR^{1^*})_{3-y} \qquad (I),$$

mit nicht wasserlöslichen Alkyltrialkoxysilanen der allgemeinen Formel IIa

$$R^2 - Si\,(OR^{1^{**}})_3 \qquad (IIa)$$

und/oder nicht wasserlöslichen Dialkyldialkoxysilanen der allgemeinen Formel III

$$AA' - Si\,(OR^{1^{***}})_2 \qquad (III),$$

und/oder Mischungen aus nicht wasserlöslichen Alkyltrialkoxysilanen und Dialkyldialkoxysilanen der allgemeinen Formeln II und III,

wobei R eine aminofunktionelle organische Gruppe ist,

$R^1$, $R^{1*}$, $R^{1**}$ und $R^{1***}$ einen Methyl- oder Ethyl-Rest,

$R^2$ einen linearen oder cyclischen oder verzweigten Alkyl-Rest mit 1 bis 8 C-Atomen,

A einen unverzweigten oder verzweigte Alkyl-Rest mit 1 bis 3 C-Atomen und

A' einen unverzweigten oder verzweigten Alkyl-Rest mit 1 bis 3 C-Atomen darstellen und

$$0 \leq y \leq 1 \text{ ist,}$$

- Versetzen des Gemisches mit Wasser, und
- Einstellen des pH-Wertes der Reaktionsmischung auf einen Wert zwischen 1 und 8 und
- Entfernen des bereits vorhandenen und/oder bei der Umsetzung entstandenen Alkohols oder

erhältlich durch

- Mischen von Q Molen wasserlöslicher Aminoalkylalkoxysilane der allgemeinen Formel I

$$R - Si\,(R^1)_y\,(OR^{1^*})_{3-y} \qquad (I),$$

mit M Molen nicht wasserlöslicher Alkylalkoxysilane der allgemeinen Formel IIb

$$R^3 - Si\,(OR^{1**})_3 \qquad \text{(IIb)},$$

wobei R eine aminofunktionelle organische Gruppe ist,

$R^1$, $R^{1*}$ und $R^{1**}$ einen Methyl- oder Ethyl-Rest und

$R^3$ einen linearen oder cyclischen oder verzweigten Alkylrest mit 1 bis 6 C-Atomen oder eine Ureido-Alkylgruppe der allgemeinen Formel IV

$$NH_2 - CO - NH - (CH_2)_b - \text{, mit } 1 \le b \le 6, \qquad \text{(IV)}$$

darstellt und
$0 \le y \le 1$ ist,

- in dem molaren Verhältnis $0 < M/Q \le 2$,
- Versetzen des Gemisches mit Wasser,
- Einstellen des pH-Wertes der Reaktionsmischung auf einen Wert zwischen 1 und 8 und
- Entfernen des bereits vorhandenen und/oder bei der Umsetzung entstandenen Alkohols oder

erhältlich durch

- Mischen wasserlöslicher Organosilane der allgemeinen Formel V

$$H_2N(CH_2)_f(NH)_g(CH_2)_i - Si\,(CH_3)_h\,(OR^0)_{3-h} \qquad \text{(V)},$$

worin $0 \le f \le 6$, g = 0 falls f = 0, g = 1 falls f > 1, $0 \le i \le 6$,$0 \le h \le 1$ und $R^0$ eine Methyl-, Ethyl-, Propyl- oder Isopropyl-Gruppe sind,

mit wasserlöslichen, jedoch in wässrigem Medium nicht stabilen

Organosilanen der allgemeine Formel VI

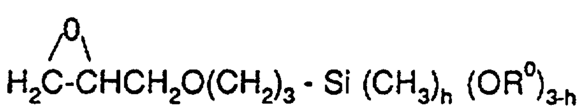

(VI),

worin $0 \le h \le 1$ ist und $R^0$ einen Methyl-, Ethyl-, Propyl- oder Isopropyl-Rest darstellt,
und/oder der allgemeinen Formel VII

$$H_2C{=}CR'{-}COO(CH_2)_3 - Si\,(CH_3)_h\,(OR^0)_{3-h} \qquad \text{(VII)},$$

worin $0 \le h \le 1$ ist, $R^0$ einen Methyl-, Ethyl-, Propyl- oder Isopropyl-Rest und R' einen Methyl- oder Wasserstoff-Rest darstellen,
und ein nicht wasserlösliches Organosilan der allgemeinen Formel VIII

$$R'' - Si\,(CH_3)_h\,(OR^0)_{3-h} \qquad (VIII),$$

worin $0 \leq h \leq 1$ ist, $R^0$ einen Methyl-, Ethyl-, Propyl- oder Isopropyl-Rest und R" einen linearen, verzweigten oder cyclischen Kohlenwasserstoff-Rest mit 1 bis 8 C-Atomen darstellen,

- in dem molaren Verhältnis $M = a / (b + c + d)$,
  wobei a die Summe der Molzahlen der Organosilane gemäß Formel V, b die Summe der Molzahlen der Organosilane gemäß Formel VI sowie c die Summe der Molzahlen der Organosilane gemäß Formel VII und d die Summe der Molzahlen der Organosilane gemäß Formel VIII sind, mit $0 \leq M \leq 3$, insbesondere für M gleich 0 mit a gleich 0 und/oder c gleich d gleich 0 und $b \leq 1$ sowie vorzugsweise $0{,}5 \leq M \leq 3$,

- Versetzen des Gemisches mit Wasser/Säure-Gemisch,

- Einstellen des pH-Wertes der Reaktionsmischung auf einen Wert zwischen 1 und 8 und

- Entfernen des bereits vorhandenen und/oder bei der Umsetzung entstandenen Alkohols.

**2.** Verfahren zur Herstellung der Perlglanzpigmente nach Anspruch 1, **dadurch gekennzeichnet, daß** man das mit Metalloxiden beschichtete Substrat in Wasser suspendiert, in einer ersten Stufe durch Zusatz geeigneter Zubereitungen, beispielsweise wäßriger Salzlösungen oder organischer Metallverbindungen von mindestens 2 Elementen aus der Gruppe Silicium, Aluminium, Cer, Titan und Zirkonium, die entsprechenden Oxidhydrate entweder gleichzeitig oder nacheinander bei einer Temperatur von 30 bis 100 °C, vorzugsweise 40 bis 75 °C, ausfällt, wobei die Oxidhydrate von Silicium und Aluminium bei einem pH-Wert von 5 bis 9 und die Oxidhydrate von Cer und Zirkonium bei einem pH-Wert von 3 bis 7 auf das Substrat aufgefällt werden und durch gleichzeitige Zugabe von Säure oder Lauge oder einem geeigneten Puffersystem der pH-Wert konstant gehalten wird und in einer zweiten Stufe bei einem pH-Wert von 3 bis 8 das oligomere Silansystem zusetzt, das auf der Pigmentoberfläche gebunden wird und anschließend das Pigment abtrennt, mit vollentsalztem Wasser wäscht und bei 80° bis 160 °C, vorzugsweise 120° bis 160 °C, trocknet.

**3.** Verfahren nach Anspruch 6, **dadurch gekennzeichnet, daß** als organische Metallverbindung ein Ester der allgemeinen Formel X

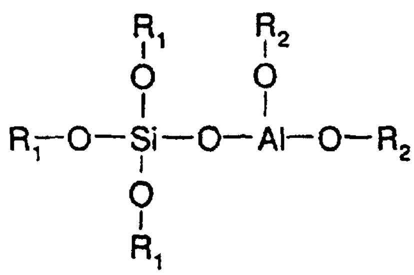

(X)

worin $R_1$ und $R_2$ gleich oder verschieden sind und eine Alkylgruppe mit 1 bis 8 Kohlenstoffatomen darstellen, eingesetzt wird.

**4.** Verfahren nach Anspruch 3, **dadurch gekennzeichnet, daß** monomeres Silan zusätzlich oder an Stelle des oligomeren Silansystems eingesetzt wird.

**5.** Verfahren nach Anspruch 4, **dadurch gekennzeichnet, daß** das monomere Silan
ein Alkyltrialkoxysilan, ein Dialkyldialkoxysilan, ein Glycidyloxyalkylalkoxysilan, ein Methacryloxyalkylalkoxysilan, ein Vinylalkoxysilan oder ein Gemisch derselben ist.

**6.** Verwendung der erfindungsgemäßen Pigmente nach Anspruch 1 zur Pigmentierung von Lacken, Druckfarben, Kunststoffen und Kosmetika.

7. Lacke, Farben, Kunststoffe und Kosmetika, die mit einem Perlglanzpigment nach Anspruche 1 pigmentiert sind.

## Claims

1. Modified pearl lustre pigments for waterborne coating systems, based on a platelet-form substrate coated with metal oxides and, located on the topmost metal oxide layer, a top layer consisting of at least two oxides and/or mixed oxides from the group consisting of silicon dioxide, aluminium oxide, cerium oxide, titanium oxide and zirconium oxide, and of a water-based oligomeric silane system which is essentially free from organic solvents, has a flash point of more than 70° and releases less than 5% by weight of alcohols by hydrolysis on dilution with water, obtainable by

- mixing water-soluble aminoalkylalkoxysilanes of the general formula I

$$R - Si (R^1)_y (OR^{1*})_{3-y} \quad (I),$$

with water-insoluble alkyltrialkoxysilanes of the general formula IIa

$$R^2 - Si (OR^{1**})_3 \quad (IIa),$$

and/or water-insoluble dialkyldialkoxysilanes of the general formula III

$$AA' - Si (OR^{1***})_2 \quad (III),$$

and/or mixtures of water-insoluble alkyltrialkoxysilanes and dialkyldialkoxysilanes of the general formulae II and III,

where R is an amino-functional organic group,

$R^1$, $R^{1*}$, $R^{1**}$ and $R^{1***}$ are a methyl or ethyl radical,

$R^2$ is a linear or cyclic or branched alkyl radical having 1 to 8 carbon atoms,

A is an unbranched or branched alkyl radical having 1 to 3 carbon atoms and

A' is an unbranched or branched alkyl radical having 1 to 3 carbon atoms, and

$$0 \leq y \leq 1,$$

- adding water to the mixture and
- adjusting the pH of the reaction mixture to between 1 and 8, and
- removing the alcohol which is already present and/or has been produced in the reaction, or obtainable by
- mixing Q moles of water-soluble aminoalkylalkoxysilanes of the general formula I

$$R - Si (R^1)_y (OR^{1*})_{3-y} \quad (I),$$

with M moles of water-soluble alkylalkoxysilanes of the general formula IIb

$$R^3 - Si\,(OR^{1**})_3 \qquad \text{(IIb)},$$

where R is an amino-functional organic group,

$R^1$, $R^{1*}$ and $R^{1**}$ are a methyl or ethyl radical and

$R^3$ is a linear or cyclic or branched alkyl radical having 1 to 6 carbon atoms or a ureido alkyl group of the general formula IV

$$NH_2 - CO - NH - (CH_2)_b -, \text{ where } 1 \leq b \leq 6, \qquad \text{(IV)}$$

and

$$0 \leq y \leq 1,$$

- in the molar ratio $0 < M/Q \leq 2$,
- adding water to the mixture,
- adjusting the pH of the reaction mixture to between 1 and 8, and
- removing the alcohol which is already present and/or has been produced in the reaction, or obtainable by
- mixing water-soluble organosilanes of the general formula V

$$H_2N(CH_2)_f(NH)_g(CH_2)_i - Si\,(CH_3)_h\,(OR^0)_{3-h} \qquad \text{(V)},$$

in which $0 \leq f \leq 6$, $g = 0$ if $f = 0$, $g = 1$ if $f > 1$, $0 \leq i \leq 6$, $0 \leq h \leq 1$ and $R^0$ is a methyl, ethyl, propyl or isopropyl group, with water-soluble organosilanes, which are nevertheless unstable in the aqueous medium, of the general formula VI

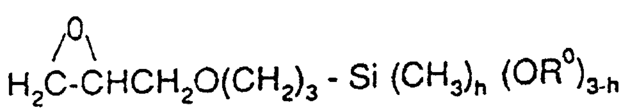

(VI),

in which $0 \leq h \leq 1$ and $R^0$ is a methyl, ethyl, propyl or isopropyl radical,
and/or of the general formula VII

$$H_2C{=}CR'\text{-}COO(CH_2)_3 - Si\,(CH_3)_h\,(OR^0)_{3-h} \qquad \text{(VII)},$$

in which $0 \leq h \leq 1$, $R^0$ is a methyl, ethyl, propyl or isopropyl radical and R' is a methyl radical or hydrogen, and a water-insoluble organosilane of the general formula VIII

$$R'' - Si\,(CH_3)_h\,(OR^0)_{3-h} \qquad \text{(VIII)},$$

in which $0 \leq h \leq 1$, $R^0$ is a methyl, ethyl, propyl or isopropyl radical and R'' is a linear, branched or cyclic hydrocarbon radical having 1 to 8 carbon atoms,

- in the molar ratio $M = a/(b+c+d)$,

where a is the sum of the mole fractions of the organosilanes according to formula V, b is the sum of the mole fractions of the organosilanes according to formula VI and c is the sum of the mole fractions of the organosilanes according to formula VII and d is the sum of the mole fractions of the organosilanes according to formula VIII, with $0 \leq M \leq 3$, especially for $M = 0$ with $a = 0$ and/or $c = d = 0$ and $b \leq 1$ and also, preferably $0.5 \leq M \leq 3$,

- adding a water/acid mixture to the mixture,

- adjusting the pH of the reaction mixture to between 1 and 8, and

- removing the alcohol which is already present and/or has been produced in the reaction.

**2.** Process for the preparation of the pearl lustre pigments according to Claim 1, **characterized in that** the metal oxide-coated substrate is suspended in water, in a first step the corresponding oxide hydrates are precipitated, either simultaneously or in succession, at a temperature of from 30 to 100°C, preferably from 40 to 75°C, by the addition of suitable formulations, for example aqueous salt solutions or organic metal compounds of at least two elements from the group consisting of silicon, aluminium, cerium, titanium and zirconium, the oxide hydrates of silicon and aluminium being precipitated at a pH of from 5 to 9 and the oxide hydrates of cerium and zirconium being precipitated at a pH of from 3 to 7 onto the substrate, and the pH is kept constant by simultaneous addition of acid or alkali or an appropriate buffer system, and in a second step at a pH of from 3 to 8 the oligomeric silane system is added which is bound on the pigment surface, and subsequently the pigment is separated off, washed with deionised water and dried at from 80 to 160°C, preferably from 120 to 160°C.

**3.** Process according to Claim 2, **characterized in that** the organometallic compound employed is an ester of the general formula X

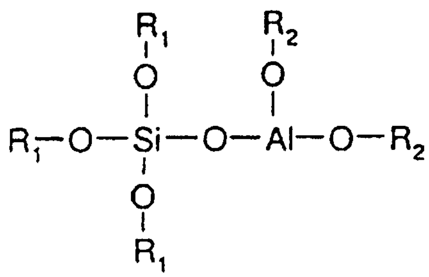

(X)

in which $R_1$ and $R_2$ are identical or different and are an alkyl group having 1 to 8 carbon atoms.

**4.** Process according to Claim 3, **characterized in that** the monomeric silane is employed in addition to or in place of the oligomeric silane system.

**5.** Process according to Claim 4, **characterized in that** the monomeric silane is an alkyltrialkoxysilane, a dialkyldialkoxysilane, a glycidyloxyalkylalkoxysilane, a methacryloxyalkylalkoxysilane, a vinylalkoxysilane or a mixture thereof.

**6.** Use of the pigments according to Claim 1 for pigmenting coating materials, printing inks, plastics and cosmetics.

**7.** Coating materials, inks, plastics and cosmetics pigmented with a pearl lustre pigment according to Claim 1.

**Revendications**

**1.** Pigments nacrés modifiés pour systèmes de peintures à l'eau, à base d'un substrat en paillettes enrobé d'oxydes métalliques et d'une couche de recouvrement se trouvant sur la couche d'oxyde métallique supérieure, la couche de recouvrement étant constituée d'au moins deux oxydes et/ou oxydes mélangés et/ou oxydes mixtes choisis dans le groupe constitué par le dioxyde de silicium, l'oxyde d'aluminium, l'oxyde de cérium, l'oxyde de titane et

l'oxyde de zirconium, et d'un système de silane oligomère à base aqueuse, qui est pratiquement exempt de solvants organiques, a un point d'inflammation de plus de 70°C et, lors de la dilution avec de l'eau, libère moins de 5 % en poids d'alcools par hydrolyse, pouvant être obtenu par

- mélange d'aminoalkylalcoxysilanes hydrosolubles de formule générale I

$$R\text{-}Si(R^1)_y(OR^{1*})_{3-y} \quad (I)$$

avec des alkyltrialcoxysilanes non hydrosolubles de formule générale IIa

$$R^2\text{-}Si(OR^{1**})_3 \quad (IIa)$$

et/ou des dialkyldialcoxysilanes non hydrosolubles de formule générale III

$$AA'\text{-}Si(OR^{1***})_2 \quad (III)$$

et/ou des mélanges d'alkytltrialcoxysilanes et de dialkyldialcoxysilanes non hydrosolubles de formules générales II et III,

R représentant un groupe organique à fonction amino,
$R^1$, $R^{1*}$, $R^{1**}$ et $R^{1***}$ représentant le groupe méthyle ou éthyle,
$R^2$ représentant un radical alkyle linéaire ou cyclique ou ramifié ayant de 1 à 8 atomes de carbone,
A représentant un radical alkyle ramifié ou non ramifié ayant de 1 à 3 atomes de carbone et
A' représentant un radical alkyle ramifié ou non ramifié ayant de 1 à 3 atomes de carbone

$$0 \le y \le 1,$$

- addition d'eau au mélange, et
- ajustement du pH du mélange réactionnel à une valeur comprise entre 1 et 8 et
- élimination de l'alcool déjà présent et/ou formé lors de la réaction, ou pouvant être obtenu par
- mélange de Q moles d'aminoalkylalcoxysilanes hydrosolubles de formule générale I

$$R\text{-}Si(R^1)_y(OR^{1*})_{3-y} \quad (I)$$

avec M moles d'alkylalcoxysilanes non hydrosolubles de formule générale IIb

$$R^3\text{-}Si(OR^{1**})_3 \quad (IIb)$$

R étant un groupe organique à fonction amino,
$R^1$, $R^{1*}$ et $R^{1**}$ représentant le groupe méthyle ou éthyle, et
$R^3$ représentant un radical alkyle linéaire ou cyclique ou ramifié ayant de 1 à 6 atomes de carbone, ou un groupe uréido-alkyle de formule générale IV

$$NH_2\text{-}CO\text{-}NH\text{-}(CH_2)_b\text{, où } 1 \le b \le 6 \quad (IV)$$

et
$0 \le y \le 1$,

- dans le rapport molaire $0 < M/Q \le 2$,
- addition d'eau au mélange,

- ajustement du pH du mélange réactionnel à une valeur comprise entre 1 et 8 et
- élimination de l'alcool déjà présent et/ou formé lors de la réaction, ou pouvant être obtenu par
- mélange d'organosilanes hydrosolubles de formule générale V

$$H_2N(CH_2)_f(NH)_g(CH_2)_i\text{-}Si(CH_3)_h(OR^0)_{3-h} \qquad (V)$$

dans laquelle $0 \leq f \leq 6$, $g \leq 0$ lorsque $f \leq 0$, $g \leq 1$ lorsque $f > 1$, $0 \leq i \leq 6{,}0 \leq h \leq 1$ et $R^0$ représente le groupe méthyle, éthyle, propyle ou isopropyle, avec des organosilanes hydrosolubles mais instables en milieu aqueux, de formule générale VI

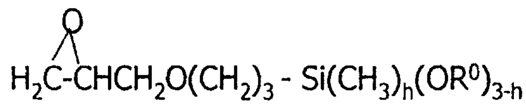 (VI)

dans laquelle $0 \leq h \leq 1$ et $R^0$ représente le groupe méthyle, éthyle, propyle ou isopropyle, et/ou de formule générale VII

$$H_2C{\leq}CR'\text{-}COO(CH_2)_3\text{ - }Si(CH_3)_h(OR^0)_{3-h} \qquad (VII)$$

dans laquelle $0 \leq h \leq 1$, $R^0$ représente le groupe méthyle, éthyle, propyle ou isopropyle et R' représente un atome d'hydrogène ou le groupe méthyle,
et un organosilane non hydrosoluble de formule générale VIII

$$R''\text{-}Si(CH_3)_h(OR^0)_{3-h} \qquad (VIII)$$

dans laquelle $0 \leq h \leq 1$, $R^0$ représente le groupe méthyle, éthyle, propyle ou isopropyle et R" représente un radical hydrocarboné linéaire, ramifié ou cyclique ayant de 1 à 8 atomes de carbone,

- dans le rapport molaire $M \leq a/(b+c+d)$,

  a étant la somme des nombres de moles des organosilanes de formule V,
  b étant la somme des nombres de moles des organosilanes de formule VI et
  c étant la somme des nombres de moles des organosilanes de formule VII et
  d étant la somme des nombres de moles des organosilanes de formule VIII, avec $0 \leq M \leq 3$, en particulier pour $M \leq 0$ avec $a \leq 0$ et/ou $c \leq d \leq 0$ et $b \leq 1$ ainsi que, de préférence, $0{,}5 \leq M \leq 3$,

- addition d'un mélange d'acide et d'eau au mélange,
- ajustement du pH du mélange réactionnel à une valeur comprise entre 1 et 8 et
- élimination de l'alcool déjà présent et/ou formé lors de la réaction.

**2.** Procédé pour la préparation des pigments nacrés selon la revendication 1, **caractérisé en ce qu'**on met en suspension dans de l'eau le substrat enrobé d'oxydes métalliques, dans une première étape, par addition de préparations appropriées, par exemple, de solutions aqueuses de sels ou de composés organométalliques d'au moins 2 éléments choisis parmi le silicium, l'aluminium, le cérium, le titane et le zirconium, les hydrates d'oxydes correspondants se déposent, soit simultanément, soit successivement, à une température de 30 à 100°C, de préférence, de 40 à 75°C, les hydrates d'oxydes du silicium et de l'aluminium étant précipités sur le substrat à un pH de 5 à 9 et les hydrates d'oxydes du cérium et du zirconium l'étant à un pH de 3 à 7, et en maintenant le pH constant par addition simultanée d'acide ou de solution alcaline ou d'un système tampon convenable, et, dans une deuxième étape, on ajoute le système de silane oligomère, à un pH de 3 à 8, qui se fixe à la surface du pigment, et le pigment est ensuite lavé avec de l'eau totalement déminéralisée et séché à 80-160°C, de préférence, à 120-160°C.

**3.** Procédé selon la revendication 2, **caractérisé en ce qu'**on utilise comme composé organométallique un ester de

formule générale X

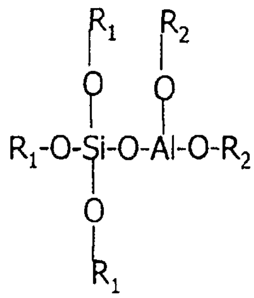

(X)

dans laquelle $R_1$ et $R_2$ sont identiques ou différents et représentent un groupe alkyle ayant de 1 à 8 atomes de carbone.

**4.** Procédé selon la revendication 3, **caractérisé en ce qu'**on utilise un silane monomère en plus ou au lieu du système de silane oligomère.

**5.** Procédé selon la revendication 4, **caractérisé en ce que** le silane monomère est
un alkyltrialcoxysilane, un dialkyldialcoxysilane, un glycidyloxyalkylalcoxysilane, un méthacryloxyalkylalcoxysilane, un vinylalcoxysilane ou un mélange de ceux-ci.

**6.** Utilisation des pigments selon la revendication 1, pour la pigmentation de peintures, encres d'imprimerie, matières plastiques et cosmétiques.

**7.** Peintures, encres, matières plastiques et cosmétiques, qui sont pigmentés avec un pigment nacré selon la revendication 1.